# EUROPEAN PATENT APPLICATION

(11) **EP 2 799 028 A1**
(43) Date of publication of application: **05.11.2014**
(21) Application number: 12863187.6
(22) Date of filing: 21.12.2012
(51) Int. Cl.: A61B 19/00, A61B 18/00

(54) **ORGANISM SIMULATION PHANTOM AND CALIBRATION DEVICE**

(30) Priority: 28.12.2011 JP 2011287368
(71) Applicant: Hitachi, Ltd., Tokyo 100-8280 (JP)
(72) Inventor: KAWABATA, Kenichi, Chiyoda-ku, Tokyo 100-8280 (JP); ASAMI, Rei, Chiyoda-ku, Tokyo 100-8280 (JP)
(74) Representative: Strehl Schübel-Hopf & Partner
(86) International application number: PCT/JP2012/083191
(87) International publication number: WO 2013/099787

(57) **Abstract**

A tissue mimicking phantom for ultrasonic treatment and a calibration device of an ultrasonic treatment device using the phantom are aimed to be provided. In order to dissolve the above-mentioned problems, the tissue mimicking phantom in accordance with the present invention is characterized by including an indicator agent which is denatured by an increase in temperature and which simulates an ultrasonic treatment effect, and a denaturation sensitivity controlling agent which is a different component from that of the indicator agent, which serves as a nucleus of cavitation at the time of ultrasonic irradiation, and which supports the increase in temperature and the denaturation of the indicator agent. The configuration makes it possible to obtain an tissue mimicking phantom which resolves the shortage of sensitivity, and which has excellent stability.

## Description

### Technical Field

The present invention relates to a phantom for displaying a treatment area of ultrasonic wave radiated from an ultrasonic device used for measurement, diagnosis, treatment, and the like, and to a device for carrying out calibration of the ultrasonic device.

### Background Art

Recently, in treatment of diseases, Quality of Life of a patient who underwent operation has been considered to be important. Even in such heavy diseases as cancers, social needs for treatment methods with lower invasiveness than conventionally have been required. Currently, low invasive treatment mainly used clinically includes treatment such as endoscopic operation, laparoscopic operation, which includes insertion of a tubular guide into a body, or treatment such as radio frequency ablation treatment, which includes insertion of a needle-like treatment device into a body, any of which are accompanied with invasiveness of devices. On the contrary, ultrasonic waves can be converged in a region having a size of 1 cm x 1 cm or less in a body from the outside of the body without inserting a device into the body, based on the relation between the wavelength and the attenuation in the body. By using the characteristics, clinical applications of low invasive ultrasonic treatment methods have been started. Ultrasonic treatment which is the most used clinically at present is High Intensity Focused Ultrasonic (HIFU) treatment subjected to uterine fibroid and breast cancer, which ablates an affected site tissue by raising a temperature of the affected site by irradiation with HIFU to at temperature equal to or higher than a coagulation temperature of protein for several seconds.

In treatment using an ultrasonic wave, since an ultrasonic wave generator is not brought into contact with a treatment region, it is necessary to monitor a region at which treatment is carried out by using a diagnostic imaging device, or the like. Furthermore, in order to carry out selective treatment more reliably, in addition to the monitoring, it is also important to schedule a treatment plan in advance, and to control the amount of ultrasonic waves so that the treatment region is irradiated with an appropriate amount of ultrasonic waves and that regions other than the treatment region is not irradiated with an inappropriately excessive amount of ultrasonic waves.

Important steps of the treatment plan in the ultrasonic treatment include verification of whether or not a device in which setting for treatment is carried out works as expected. Such verification can be achieved before application to a human body by irradiating an ultrasonic phantom (tissue mimicking phantom), which has been configured so as to be able to simulate a living body, to display the extent and range of the biological effect generated by ultrasonic irradiation inside thereof, with ultrasonic wave, and observing and analyzing the results.

As the above-mentioned ultrasonic phantom, one for visualizing not energy of an ultrasonic wave itself but a secondary effect generated by an ultrasonic wave is mainly used. Examples thereof include a phantom shown in NPL 1, which uses soluble protein as an indicator agent and detects temperature rise due to irradiation with an ultrasonic wave. This phantom uses the phenomena that when protein undergoes heat denaturation, the protein is coagulated and molecules are aggregated to each other, and scattering intensity is increased to cause optical change as compared with before the denaturation, in particular, whitening occurs.

### Citation List

### Non-Patent Literature

NPL 1: C Lafon et al. Proc. IEEE Ultrasonics Symposium pp. 1295-1298 (2001)

### Summary of Invention

### Technical Problem

A conventionally used tissue mimicking phantom for HIFU treatment detects the protein appearance change from translucent to opaque due to denaturation by visual check or an optical technique. However, there is a problem that the phantom cannot carry out controlling of ultrasonic intensity in which the optical changeoccurs, as well as controlling of optical turbidity and nucleus of cavitation, independently. That is because determination of the strength of ultrasonic intensity necessary for optical change is carried out inclusively based on the effect as a criteria of buffers such as lower alcohol and tris-(hydroxymethyl)-aminomethane (hereinafter, which is abbreviated as "Tris"), or the like. Specific buffers such as lower alcohol and Tris change a three-dimensional structure of bovine serum albumin and deteriorates dissolution stability in a solution. Therefore, albumin forms an aggregated body, but such an aggregated body is in a semi-denatured state. When temperature is increased due to ultrasonic irradiation or the like, the aggregated body of albumin is more susceptible to denaturation as compared with a simple substance of albumin. Such an effect that denaturation of protein easily occurs by ultrasonic irradiation is remarkably found in Tris. However, a protein solution including an aggregated body has higher turbidity than a solution in a state in which protein completely dissolved, which poses a problem when a phantom having a particularly large size is prepared.

Furthermore, the protein in such an aggregated body state works as nucleus of acoustic cavitation mentioned below, but since the phantom includes a region in which an aggregated body is generated and a region in which an aggregated body is not generated, nuclei are scattered, and as a result, denaturation is not generated uniformly in the phantom. Furthermore, since Tris or lower alcohol is a low molecule, there is a problem that Tris or lower alcohol has a property of easily passing through a network structure of hydrogel as base material of an tissue mimicking phantom, so that Tris or lower alcohol easily leaks out from the phantom. Therefore, as in, for example, a phantom-bed integral type large ultrasonic irradiation device, when it is necessary to use a phantom in a state in which it is brought into direct contact with an ultrasonic device, the component concentration is changed over time. Therefore, it is highly likely that properties as the phantom may be deteriorated.

Note here that an acoustic cavitation denotes a phenomenon in which minutes air bubbles are generated from a substance as a nucleus generated by irradiation with respect to liquid, organisms, or the like, with ultrasonic waves, is grown and finally collapsed by ultrasonic vibration.

### Solution to Problem

In order to solve the above-mentioned problem, an tissue mimicking phantom in accordance with the present invention is characterized by including an indicator agent which is denatured by temperature rise to simulate the effects of ultrasonic treatment, and a denaturation sensitivity controlling agent which is a different component from the indicator agent and which serves as a nucleus of cavitation and supports the temperature rise and the denaturation of the indicator agent at the time of irradiation with ultrasonic waves.

### Advantageous Effects of Invention

According to the ultrasonic phantom of the present invention, it is possible to independently control a degree at which optical changes occur by the irradiation with ultrasonic waves, optical transparency before the irradiation with ultrasonic waves, and a degree of serving as the nucleus of cavitation at a time of ultrasonic irradiation. Calibration of the strength of an ultrasonic treatment device can be carried out more stably than conventionally possible. Furthermore, the ultrasonic phantom of the present invention can be used in a state in which the phantom is taken out from a container and is closely brought into direct contact with an ultrasonic irradiation device. Thus, there is little limitation in usage forms.

### Brief Description of Drawings

[FIG. 1] FIG. 1 is a view showing an experimental system in which verification of effects of a denaturation sensitivity controlling agent for tissue mimicking phantom is carried out in accordance with the present invention.
[FIG. 2] FIG. 2 is a view showing an experimental system in which verification of effects of the tissue mimicking phantom is carried out in accordance with the present invention.
[FIG. 3] FIG. 3 is a view showing one example of an optical image after the tissue mimicking phantom is irradiated with convergent ultrasonic wave in accordance with the present invention.
[FIG. 4] FIG. 4 is a graph showing one example of a change in a denatured region when irradiation with a focused ultrasonic wave is carried out with the intensity varied in a state in which a temperature of the tissue mimicking phantom is kept at 37°C in accordance with the present invention.
[FIG. 5] FIG. 5 is a graph showing one example of a change in a denatured region when irradiation with a focused ultrasonic wave is carried out with the intensity varied in a state in which a temperature of the tissue mimicking phantom is kept at 25°C in accordance with the present invention.
[FIG. 6] FIG. 6 is a graph showing one example of a difference in the denatured region when irradiation with a strongly focused ultrasonic wave is carried out in a state in which a temperature of the tissue mimicking phantom prepared while a serum the concentration of albumin is changed is kept at 37°C in accordance with the present invention.
[FIG. 7] FIG. 7 is a view showing an outer frame of ultrasonic phantom in accordance with one Example of the present invention.
[FIG. 8] FIG. 8 is a view showing a gel of a phantom main body of the ultrasonic phantom in accordance with one Example of the present invention.
[FIG. 9] FIG. 9 is a view showing one example of a calibration device of an ultrasonic treatment device which is used in combination of the ultrasonic phantom of the present invention.
[FIG. 10] FIG. 10 is a view showing one example of effect verification of a denaturation sensitivity controlling agent for an tissue mimicking phantom in accordance with the present invention.

### Description of Embodiments

In a conventional ultrasonic phantom, it was difficult to control the sensitivity at which denaturation occurs when irradiation with an ultrasonic wave is carried out, optical turbidity, and an effect as a nucleus of cavitation independently. This is because a component to be denatured is in a metastable state, thus improving sensitivity, with the effects of Tris and lower alcohol, when irradiation with an ultrasonic wave is carried out, but this metastable state increases optical turbidity and has an effect as a nucleus of cavitation. Furthermore, in such an existing phantom, since low molecules such as Tris are used for expressing the effects, the above-mentioned three effects easily disappear due to flowing of the low molecules such as Tris from the inside of the phantom having a gel form. Thus, the existing phantom lacks in stability over time.

From these viewpoints, we have devised a method of allowing a phantom to additionally contain material, which has a property that is substantially the same as in a state in which the component generating denaturation becomes a metastable state by the presence of Tris or the like, as a denaturation sensitivity controlling agent, in addition to the component generating denaturation when irradiation with ultrasonic waves is carried out, instead of using material which directly acts on a component, typically Tris, generating denaturation when irradiation with an ultrasonic wave is carried out. In particular, unlike conventional phantom, by using not a low molecule but a high molecule, phantom, which has taken stability over time into account, is achieved.

Specifically, the phantom is configured by including, as a denaturation sensitivity controlling agent, a high molecular compound whose turbidity in the wavelength of 600 nm when it is dispersed at 1 gram per 1 liter in a pure water and in a neutral state becomes 0.001 or more and 0.1 or less per 1 cm. The use of such a method can dissolve a problem that the turbidity of a phantom as a whole is increased when metastable state protein is used as conventionally.

As a result of study, as the high molecular compound used as the denaturation sensitivity controlling agent, we found that protein having low solubility in water, or protein whose solubility in water is lowered by pretreatment is preferred. More specifically, we have found that egg white albumin, milk albumin, globulin in general having lower solubility in water than that of serum albumin satisfy the purpose. Furthermore, regardless of the solubility in water, we have found that water-soluble protein, which was subjected to heat treatment, or ultrasonic treatment, or radiation irradiation in advance, satisfy the purpose.

Hereinafter, Test Examples and Examples of the present invention are described specifically, but the present invention is not limited to these Examples.

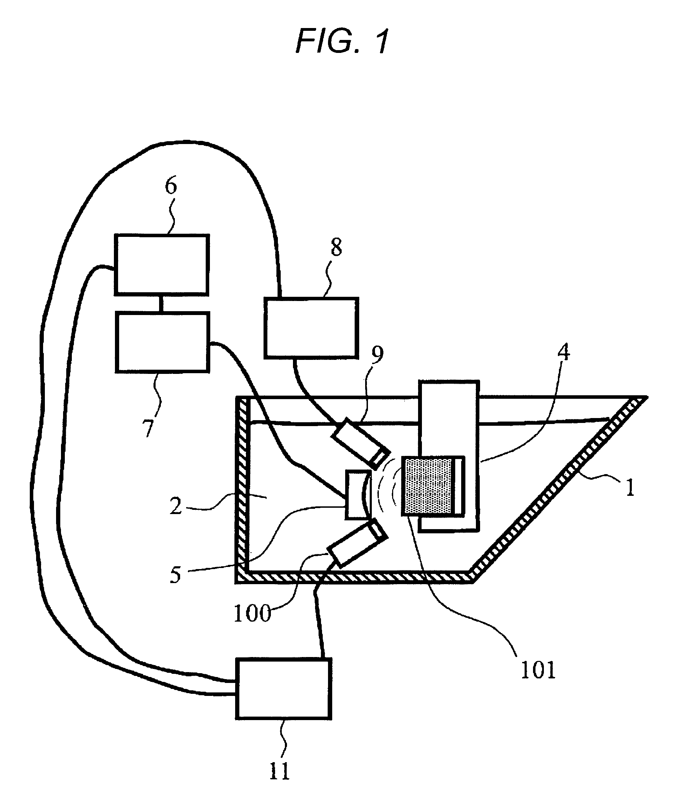

Firstly, in order to search material to be used as the denaturation sensitivity controlling agent, an effect of generating cavitation when the material is dispersed in water and irradiation with an ultrasonic wave is carried out is measured by using an experimental system shown in FIG. 1. An acrylic water tank 1 is filled with degassed water 2, the water temperature is kept at 37°C by using a water temperature adjuster and a thermometer which are not shown in the drawing. In the water tank, a sample 101 is fixed at a focus position of convergent ultrasonic wave transducer 5 by a holder 4 in a state in which the sample 101 is dispersed in water in the tank and placed in a polyethylene bag having thickness of 0.03 mm.

The transducer 5 is connected to a waveform generator 6 and signal amplifier 7. Furthermore, in order to verify the position of the sample 101, an ultrasonic diagnostic device 8 and a diagnostic probe 9 are provided in the water. Furthermore, an acoustic signal generated from the sample 101 by ultrasonic irradiation is measured by using a hydrophone 100, and the acoustic signal is stored in an oscilloscope 102. The waveform generating device 6, the ultrasonic diagnostic device 8, and the oscilloscope 102 are connected to a control oriented computer 11, and set so that the acoustic signal taken by a hydrophone 100 in synchronization with ultrasonic irradiation from the waveform generating device 6.

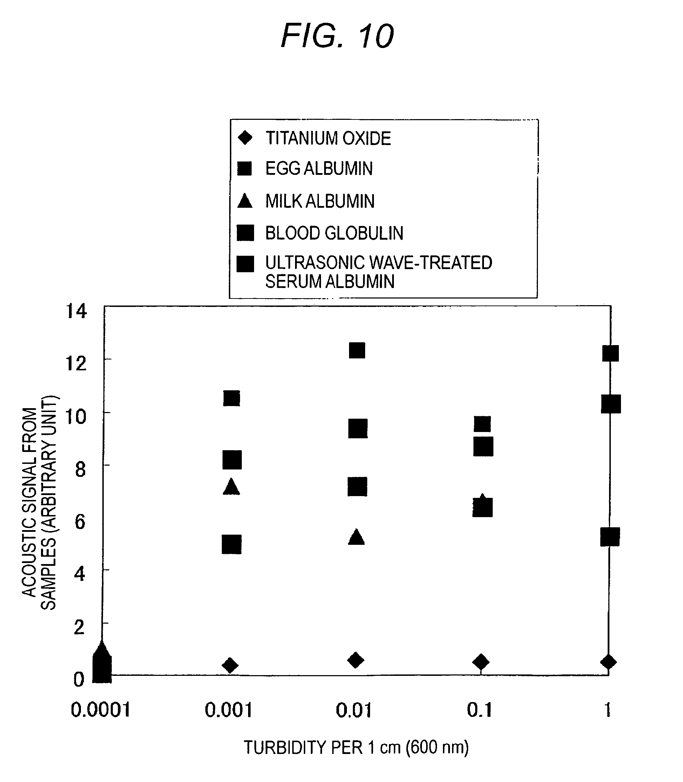

FIG. 10 shows a signal strength taken by the hydrophone 100 as an indicator of cavitation strength when a concentration of a sample is changed with respect to the turbidity in the wavelength of 600 nm as a an indicator. As the sample, titanium oxide fine particles, egg albumin, milk albumin, blood globulin, and serum albumin that had been treated in the ultrasonic washer for two minutes were used. From the drawing, an acoustic signal is returned from the samples except for titanium oxide, showing that the samples serve as the nucleus of cavitation. Furthermore, in the samples other than titanium oxide, it is shown that an effect when the turbidity is 0.001 or more.

Furthermore, each sample was prepared in a thickness of 2 cm, and a chicken breast meat piece, which had been cut in a size of 1 cm³, was disposed behind the sample. Then, when it was verified whether or not the shape of the chicken breast meat piece was able to be determined, it was shown that the shape was able to be clearly determined when the turbidity was not more than 0.1 per 1 cm.

Considering the above-mentioned experimental fact and considering that a denaturation indicator agent of the present invention is serum albumin having a concentration of about 10% and that the concentration of the denaturation sensitivity controlling agent is desirably not more than one-tenth of that of the denaturation indicator agent so that denatured regions are not spotted, it is desirable that the concentration of the denaturation sensitivity controlling agent is not more than about 1%. Thus, it is shown to be desirable that in the denaturation sensitivity controlling agent of the present invention, the turbidity in the wavelength of 600 nm is not more than 0.001 and not less than 0.1 when 10 gram per liter is dispersed.

Subsequently, an tissue mimicking phantom including serum albumin as a denaturation indicator agent which is denatured due to temperature rise and which simulates an ultrasonic treatment effect, and egg white albumin as a denaturation sensitivity controlling agent which becomes a nucleus of cavitation when irradiation with an ultrasonic wave is carried out and which controls the temperature rise and the denaturation sensitivity of the indicator agent is prepared, and the properties thereof are evaluated. The evaluation results are described.

### (1) Preparation of Phantom

All operations hereinafter were carried out at 4°C. An aqueous solution (86.5 ml) of containing 15% bovine serum albumin and 5 ml of pure water in which 0.1% egg white albumin had been dispersed and 25 ml of 40% solution of acrylamide (acrylamide : bisacrylamide = 39 : 1) were sufficiently mixed with each other, followed by degasification. Then, the mixture was poured into a rectangular parallelepiped container. While the mixture was mildly stirred with a stirrer, 5 ml of 10% solution of ammonium persulfate and 5 ml of N,N,N',N'-tetramethyl ethylenediamine were rapidly added. When they were mixed homogeneously, the stirring was stopped, a stirrer bar was removed, and a cover was put on the rectangular parallelepiped container and the container was stood still for 20 minutes. From the above-mentioned operations, substantially transparent gel was prepared and used as a tissue mimicking phantom. Furthermore, a gel which does not contain egg white albumin was prepared and used as a control phantom.

### (2) Experimental System Used for Test

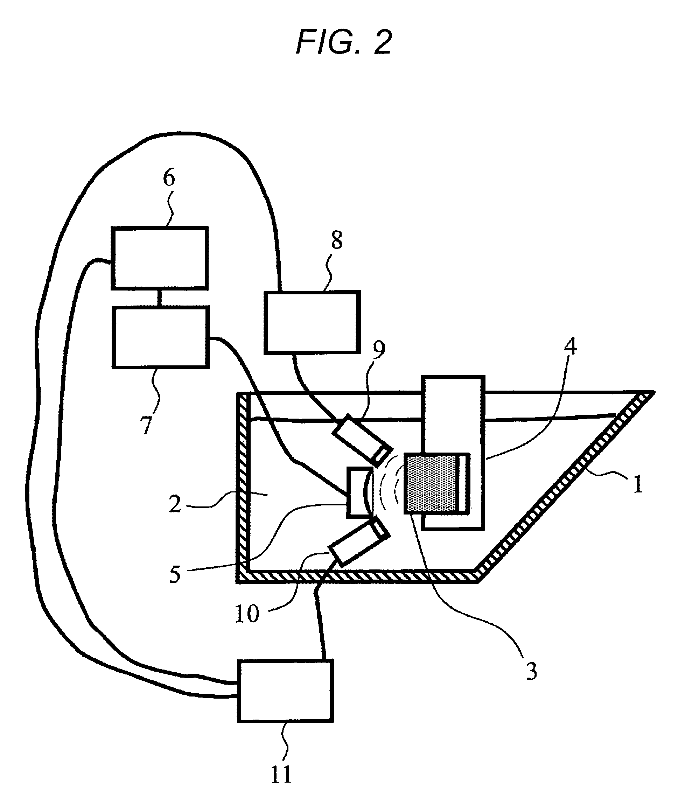

The below-mentioned tests were carried out by using an experimental system shown in FIG. 2. An acrylic water tank 1 is filled with degassed water 2, water temperature is kept at 37°C or 25°C by using a water temperature adjuster and a thermometer which are not shown in the drawing. In this water tank, the tissue mimicking phantom 3 or the control phantom prepared according to the above-mentioned phantom preparation method (1) were fixed to a focus position of convergent ultrasonic wave of the transducer 5 by the holder 4 in a state in which they were placed in a polyethylene bag having a thickness of 0.03 mm. The transducer 5 is connected to the waveform generator 6 and the signal amplifier 7. Furthermore, in order to verify the position of the phantom 3, the ultrasonic diagnostic device 8 and the diagnostic probe 9 are located in water. Furthermore, a camera 10 for observing an optical change of the phantom due to ultrasonic irradiation is disposed in a position at which a picture of the phantom 3 can be obtained. The waveform generating device 6, the ultrasonic diagnostic device 8, and the camera 10 are connected to the control oriented computer 11 and set such that a picture of the camera 10 is taken in synchronization with the ultrasonic irradiation from the waveform generating device 6.

### (3) Calculation of Denatured Region in Phantom

Calculation of a denatured region in a phantom in the below-mentioned tests was carried out by the following procedure.

### (Cutting out of Still Picture from Moving Picture)

• Portions during ultrasonic irradiation were selected from moving pictures stored in the AVI format, then they were converted into a grayscale, and then cut out into a still picture group in the BMP format.

### (Formation of Differential Image and Binarization)

• A differential image in which the brightness of the ultrasonic irradiation is subtracted from the brightness of each pixel of each still picture obtained in 1), and then, binarization processing in which pixels having finite difference of the brightness higher than the threshold obtained in the preliminary study are made to be white and pixels other than the above pixels are made to be black is carried out.

### (Determination of Rotation axis)

Pixel values of the pictures which had undergone binarization processing were multiplied in the direction of the ultrasonic irradiation, and a region showing the highest value was made to be a central axis in which denaturation occurred.

### (Integration Processing)

Integration processing is carried out assuming rotational symmetry around the above-mentioned central axis. Note here that by applying the actual distance (unit: millimeter) in the image between the neighboring pixels which had been calculated, the results of the integration processing are made into a cubic millimeter unit.

### <Text Example 1> Effects when ultrasonic irradiation is carried with acoustic intensity varied

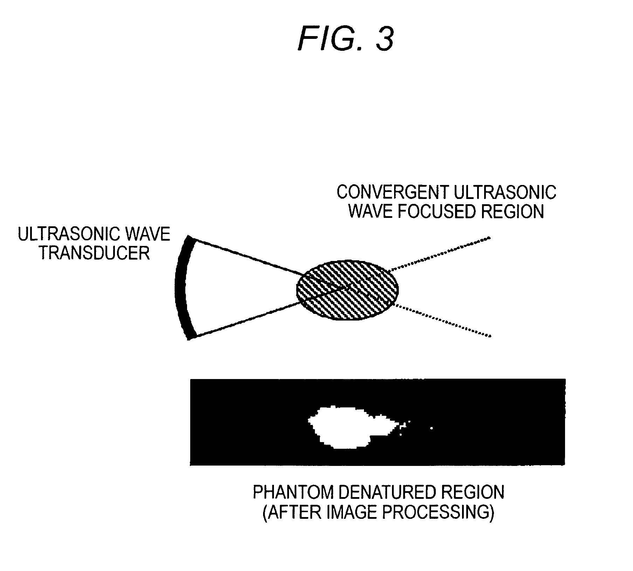

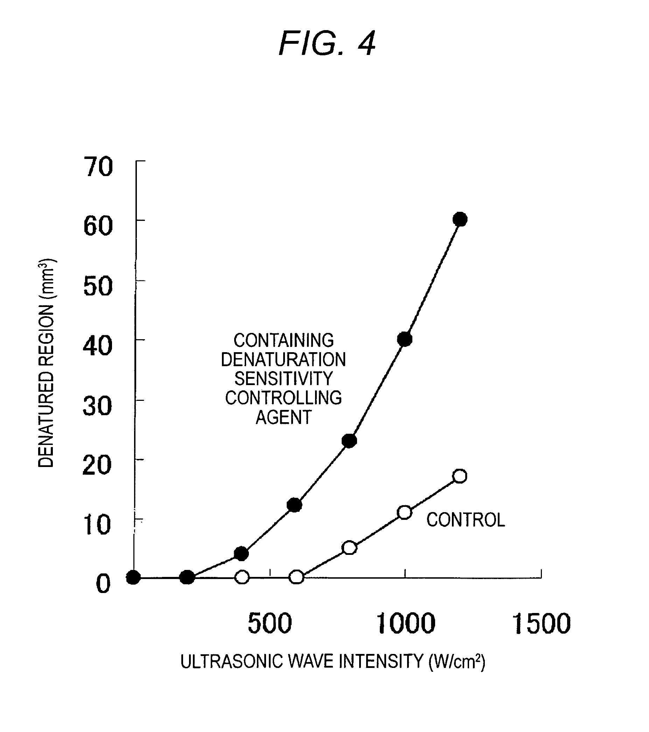

Firstly, a phantom was prepared according to the above-mentioned gel preparation method (1). An experimental system shown in FIG. 2 was used and a convergent ultrasonic wave transducer having a diameter of 48 mm and F value of 1.0 was brought into direct contact therewith in a state in which the temperature was kept at 37°C, and irradiation with an ultrasonic wave of 1.1 MHz was carried out for 15 seconds with the acoustic intensity varied from 0 to 1200 W/cm². One example of the outline of the phantom after the irradiation with an ultrasonic wave is shown in FIG. 3. FIG. 3 shows binarization by the above-mentioned processing method (3)-2). It is shown that the focal region of the ultrasonic wave becomes white in a form of a football. This is a denatured region. This denatured region is calculated in a cubic millimeter unit by the above-mentioned processing method (3), and the dependency on the ultrasonic intensity is obtained. One example of the result is shown in FIG. 4. The drawing shows the result of a phantom into which egg white albumin as a denaturation sensitivity controlling agent was filled and the result of a control phantom which does not include a denaturation sensitivity controlling agent, together. According to FIG. 4, the effect of the denaturation sensitivity controlling agent is remarkable. In the control phantom, the maximum intensity at which denaturation is not generated is 600 W/cm², while in the case including a denaturation promoter, it is lowered to 200 W/cm². Furthermore, when the ultrasonic irradiation is carried out in intensities not lower than the intensity necessary for the denaturation, in any intensities, the denatured region becomes larger when the denaturation sensitivity controlling agent is included. For example, in the intensity of 1200 W/cm², about 3.5 times larger volume is denatured. Since the visual observation becomes easier and error in carrying out quantification becomes smaller as the volume is larger, the effect of the phantom into which a denaturation sensitivity controlling agent is filled is obvious as shown in FIG. 4. Note here that the same experiment is carried out with the ultrasonic frequency varied from 1 to 6 MHz and the denaturation sensitivity controlling agent is allowed to co-exist similar to FIG. 4, it is shown that the ultrasonic intensity necessary for denaturation can be lowered. It is also shown that as the ultrasonic intensity becomes higher, the denatured region tends to be increased. Furthermore, the same was true to the case in which milk albumin, blood globulin, and ultrasonic wave-denatured serum albumin were used as the denaturation sensitivity controlling agent. Note here that the denaturation sensitivity controlling agent shows an effect when the turbidity shown in FIG. 10 is 0.001 or more, but it is used in the concentration in which the turbidity is about 0.1 or less in order to secure the optical transparency of the phantom and the uniformity in the denaturation.

### <Text Example 2> Effects when ultrasonic irradiation is carried with acoustic intensity varied (study at room temperature)

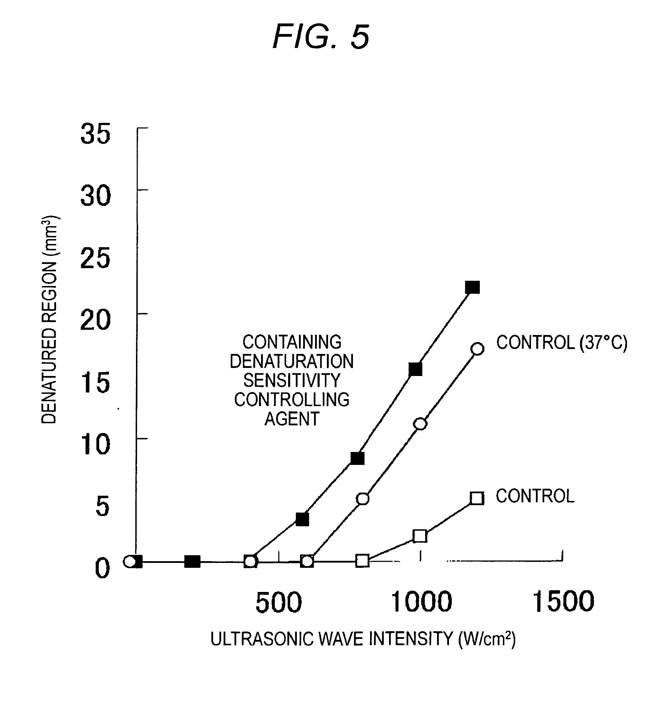

Study at room temperature assuming the use of phantom in a simple configuration without increasing temperatures was carried out. Firstly, phantom was prepared according to the above-mentioned gel preparation method (1). An experimental system shown in FIG. 2 was used and a convergent ultrasonic wave transducer having a diameter of 48 mm and F value of 1.0 was brought into direct contact therewith in a state in which the temperature was kept at 25°C, and irradiation with an ultrasonic wave of 1.1 MHz was carried out for 15 seconds with the acoustic intensity varied from 0 to 120°0 W/cm². This denatured region was calculated in a cubic millimeter unit by the above-mentioned processing method (3), and the dependency on the ultrasonic intensity was obtained. One example of the obtained result is shown in FIG. 5. The drawing shows the result of a phantom into which egg white albumin as a denaturation sensitivity controlling agent was filled, the result of the control phantom which does not include a denaturation sensitivity controlling agent, and the result of the case in which an experiment is carried out by heating the control phantom which does not include a denaturation sensitivity controlling agent at 37°C, together.

According to FIG. 5, the effect of the denaturation sensitivity controlling agent is remarkable also at room temperature (25°C). Firstly, it is shown that the ultrasonic intensity necessary for denaturation is significantly lowered to 400 W/cm² from 800 W/cm² in the control phantom (25°C). F Furthermore, when the ultrasonic irradiation is carried out in intensities not lower than the intensity necessary for the denaturation, in any intensities, the denatured region becomes larger when the denaturation sensitivity controlling agent is included. For example, in the intensity of 1200 W/cm², the volume that is about 4.5 times larger than that of the control phantom (25°C) which does not include the denaturation sensitivity controlling agent is denatured.

Since the visual observation becomes easier and error in carrying out quantification becomes smaller as the volume is larger, the effect of the phantom into which a denaturation sensitivity controlling agent is filled is obvious as shown in FIG. 5. In particular, since this study is carried out at room temperature, it is not necessary to heat the water tank for verification, a verification system and a verification operation can be simplified. Note here that when the same experiments are carried out with the ultrasonic frequency varied from 1 to 6 MHz, and the denaturation sensitivity controlling agent is allowed to co-exist similar to FIG. 5, it is shown that the ultrasonic intensity necessary for denaturation can be lowered. It is also shown that as the ultrasonic intensity becomes higher, the denatured region tends to be increased.

Furthermore, the same was true to the case in which milk albumin, blood globulin, and ultrasonic wave-denatured serum albumin were used as the denaturation sensitivity controlling agent. Note here that the denaturation sensitivity controlling agent shows an effect when the turbidity shown in FIG. 10 is 0.001 or more, but it is used in the concentration in which the turbidity is about 0.1 or less in order to secure the optical transparency of the phantom and the uniformity in the denaturation.

### <Text Example 3> Effects when ultrasonic irradiation is carried out with indicator agent concentration varied

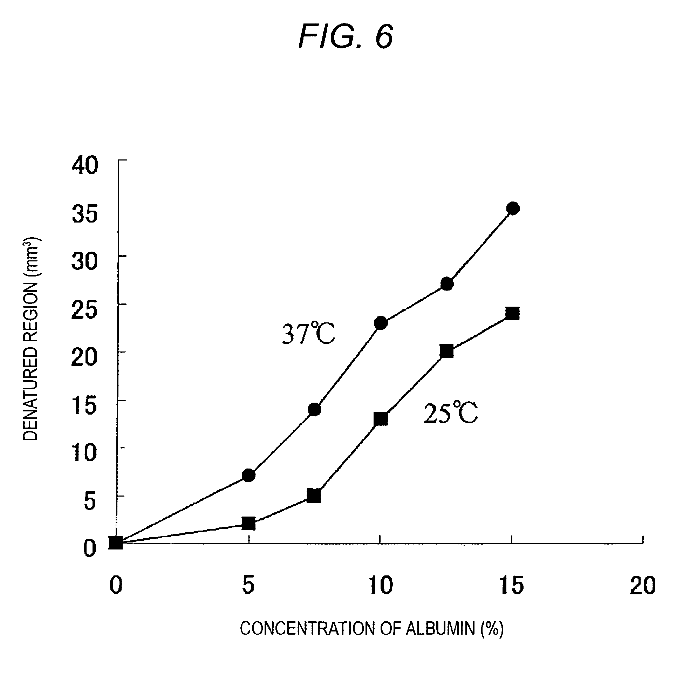

In order to verify that the size of the denatured region can be controlled by varying the concentration of the indicator agent, phantom was prepared according to the above-mentioned phantom preparation method (1) with the concentration of the serum albumin varied. An experimental system shown in FIG. 2 was used and a convergent ultrasonic wave transducer having a diameter of 48 mm and F value of 1.0 was brought into direct contact therewith in a state in which the temperature was kept at 37°C or 25°C, and irradiation with an ultrasonic wave of 1.1 MHz was carried out for 15 seconds with the acoustic intensity varied from 0 to 800 W/cm². This denatured region was calculated in a cubic millimeter unit by the above-mentioned processing method (3), and the dependency on the concentration of albumin in the phantom was obtained. One example of the obtained result is shown in FIG. 6.

According to FIG. 6, it is shown that the effect of the denaturation promoter is changed dependent upon the concentration of albumin. At both 37°C and 25°C, as the concentration of albumin is higher, the denatured region becomes larger. From this result, the phantom in accordance with the present invention can change the denatured region according to the property of the region to be simulated.

Note here that when the same experiment was carried out with the ultrasonic frequency varied from 1 to 6 MHz, similar to FIG. 6, effects were demonstrated in which as the concentration of albumin was made to be higher, the size of the denatured region was increased. Furthermore, the same was true to the case in which blood globulin and ultrasonic wave-denatured serum albumin were used as the denaturation sensitivity controlling agent. Note here that the denaturation sensitivity controlling agent shows an effect when the turbidity shown in FIG. 10 is 0.001 or more, but it is used in the concentration in which the turbidity is about 0.1 or less in order to secure the optical transparency of the phantom and the uniformity in the denaturation.

From the above-mentioned tests, the effectiveness of the tissue mimicking phantom in accordance with the present invention is shown. Hereinafter, Examples in which it is actually used are described.

### [Example 1]

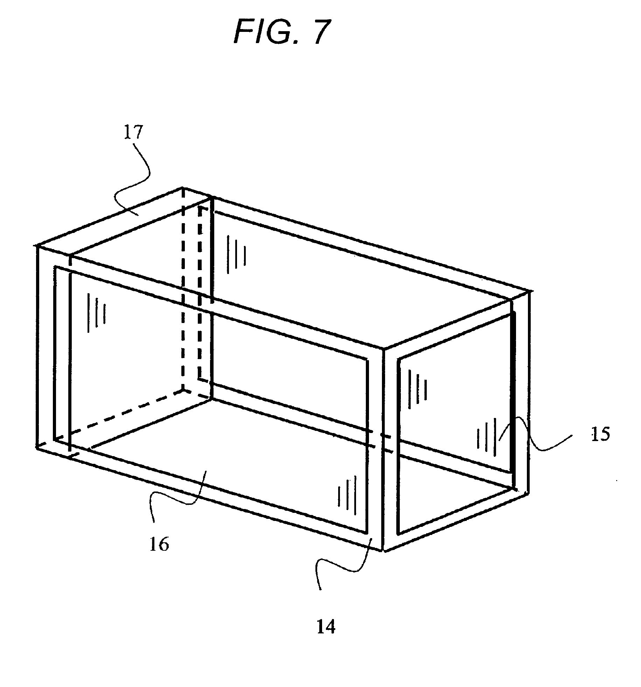

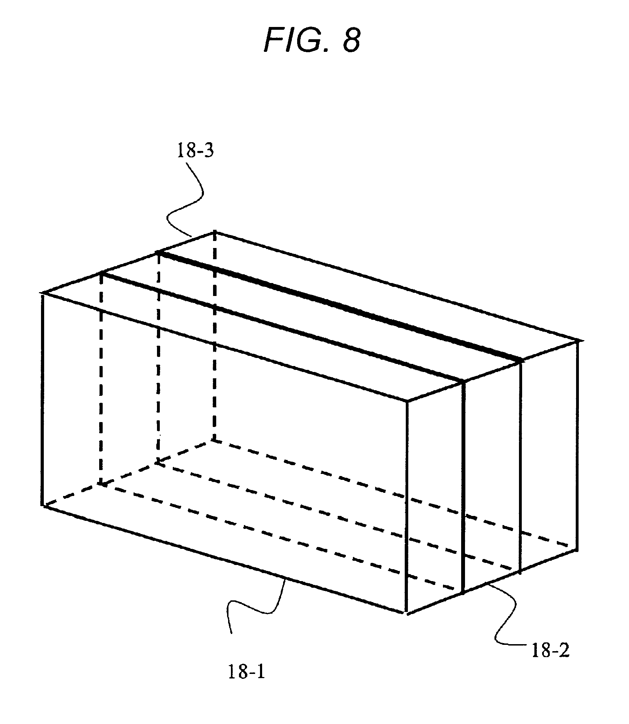

An example of a phantom for evaluating ultrasonic-wave organism action is described. Hereinafter, one Example of the present invention is described with reference to FIGS. 7 and 8. FIG. 7 shows an outer frame of the phantom. The phantom includes an outer frame main body 14, an acoustic window 15 for ultrasonic irradiation, a window 16 for observing ultrasonic irradiation results, and an ultrasonic wave antireflection layer 17. FIG. 8 is a view for showing one example of a phantom main body. The phantom main body includes three components of air bubble-mixed phantom 18-1, liquid-mixed phantom 18-2, and solid-mixed phantom 18-3, which are prepared in a state in which they are brought into close contact with each other. When the phantom is used, the phantom main body prepared according to the preparation method (1) of phantom is filled into the inside of the outer frame shown in FIG. 8.

At the use time as the phantom, an ultrasonic irradiation source to be evaluated is brought into close contact with the acoustic window 15 for ultrasonic irradiation and is irradiated with ultrasonic wave, and the result is observed from the window 16 for observing ultrasonic irradiation results. When the ultrasonic irradiation source and the acoustic window 15 for ultrasonic irradiation cannot be brought into contact with each other, irradiation with an ultrasonic wave can be carried out in a state in which the phantom and the ultrasonic irradiation source are placed into a water tank. Furthermore, irradiation can be carried out in a state in which a portion between the acoustic window 16 for ultrasonic irradiation and the ultrasonic irradiation source is filled with an acoustic coupling agent such as acoustic jelly.

Note here that in the preparation method (1), a homogeneous phantom is prepared, but the property of the phantom to be placed and used in the outer frame shown in FIG. 8 is not necessarily homogeneous. For example, as in the case as shown in FIG. 7 in which the outer frame is divided into a plurality of parts, and the air bubble-mixed phantom 18-1, the liquid-mixed phantom 18-2, and the solid-mixed phantom 18-3 are used in the parts respectively, different regions in the organism can be placed in the same frame of the phantom to be simulated. Furthermore, in the outer frame shown in FIG. 7, other than the phantom, an absorbing body or a scattering body for preventing an ultrasonic wave with which the phantom is irradiated from returning to or being reflected from the irradiation source.

### [Example 2]

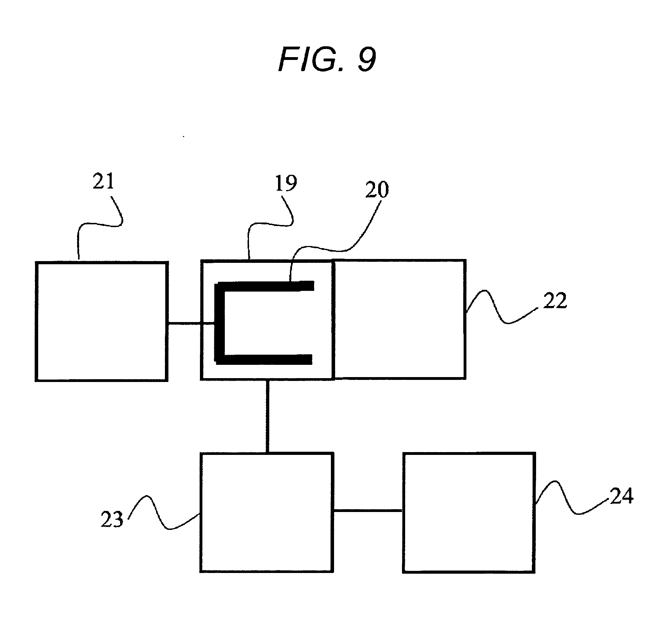

An example of a calibration device of an ultrasonic treatment device is described. Hereinafter, one Example of the present invention is described with reference to FIG. 9. The calibration device of the ultrasonic treatment device in this Example includes a phantom holding portion 19, a temperature adjusting unit 20, a temperature adjusting control unit 21, a phantom photographing unit 22, a device control unit 23, and a treatment device interface section 24.

The phantom holding portion 9 holds a phantom shown in FIG. 7, and is configured to carry out irradiation with an ultrasonic wave. The temperature adjusting unit 20 is configured to control a temperature of the phantom placed in the phantom holding portion 19 in a temperature range from 20°C to 40°C, and is controlled by the temperature adjusting control unit 21. The phantom photographing unit 22 is configured to photograph a whole phantom, and the photographed results are transferred to the device control unit 23. The device control unit 23 controls the temperature adjusting control unit 20 and the phantom photographing unit 22, and is configured to hold phantom images photographed by the phantom photographing unit 22 and to carry out an image processing such as binarization, differentiation, overlapping, and the like. The treatment device interface section 24 is connected to a treatment device, and has functions of receiving the conditions for ultrasonic irradiation from the treatment device, transferring it to the device control unit 23, and receiving the information on the phantom including, for example, the denatured region and the center position of denaturation of the phantom after ultrasonic irradiation from device control unit 23 transmits it to the treatment device.

Furthermore, when a parameter is input in advance, a function of setting information of whether or not the denatured region and the center position of the denaturation are included in the set range can be transferred to the treatment device, and a function of issuing an alarm when the obtained result is out of the set range or a function of disabling ultrasonic irradiation of the treatment device can be provided.

In carrying out the calibration device of this Example, for example, the following procedure is carried out. Firstly, in a treatment plan scheduled based on the a diagnostic image of an affected site to be treated, conditions such as an irradiation position of ultrasonic wave for treatment and irradiation time at each point are determined. In carrying out treatment, immediately before ultrasonic irradiation to the affected site, by using a calibration device including phantom according to forms and properties of the affected site of the present invention, ultrasonic irradiation is carried out in the same conditions in which treatment is carried out. When the denatured region is included in the assumed range, treatment is started. When the denatured region is not included in the assumed range, maintenance is carried out with respect to abnormality of the treatment device.

### Reference Signs List

- 1: water tank
- 2: degassed water
- 3: tissue mimicking phantom
- 4: holder
- 5: convergent ultrasonic wave transducer
- 6: waveform generator
- 7: signal amplifier
- 8: ultrasonic diagnostic device
- 9: diagnostic probe
- 10: camera
- 11: control oriented computer
- 14: phantom outer frame main body
- 15: acoustic window for ultrasonic irradiation
- 16: window for observing ultrasonic irradiation result
- 17: ultrasonic wave antireflection layer
- 18: example of tissue mimicking phantom to be filled in outer frame
- 19: phantom holding portion
- 20: temperature adjusting unit
- 21: temperature adjusting control unit
- 22: phantom photographing unit
- 23: device control unit
- 24: treatment device interface section
- 100: hydrophone
- 101: sample for measuring cavitation generation
- 102: oscilloscope

## Claims

1. A tissue mimicking phantom for ultrasonic treatment, comprising:
an indicator agent which is denatured by an increase in temperature and which simulates an ultrasonic treatment effect; and
a denaturation sensitivity controlling agent including a different component than that of the indicator agent, which serves as a nucleus of cavitation at a time of ultrasonic irradiation to support the increase in temperature and denaturation of the indicator agent.

2. A tissue mimicking phantom for ultrasonic treatment comprising:
a compound including a hydrogel as a base material, being a different component from a protein denaturation indicator drug and the indicator drug, and promoting denaturation of protein when ultrasonic irradiation is carried out.

3. The tissue mimicking phantom for ultrasonic treatment according to claim 2, wherein the compound for promoting denaturation of protein when ultrasonic irradiation is carried out is protein which has turbidity in a wavelength of 600 nm is not more than 0.001 and not less than 0.1 per 1 cm when 10 gram per 1 liter is dispersed.

4. The tissue mimicking phantom for ultrasonic treatment according to claim 3, wherein protein which is not dissolved in water is at least one protein selected from egg albumin, milk albumin, and blood globulin.

5. The tissue mimicking phantom for ultrasonic treatment according to claim 3, wherein the protein which is not dissolved in water is water-soluble protein which is denatured by thermal treatment, ultrasonic treatment, or irradiation with an X ray.
